(19)

**Europäisches Patentamt**
**European Patent Office**
**Office européen des brevets**

(11)  **EP 2 960 818 A1**

(12)  **EUROPEAN PATENT APPLICATION**

(43) Date of publication:
**30.12.2015  Bulletin 2015/53**

(51) Int Cl.:
**G06F 19/22** (2011.01)     G06F 19/24 (2011.01)

(21) Application number: **14305997.0**

(22) Date of filing: **24.06.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **INSTITUT PASTEUR**
**75015 Paris (FR)**

(72) Inventors:
• **KOSZUL, Romain**
**75011 PARIS (FR)**

• **MARBOUTY, Martial**
**91440 BURES-SUR-YVETTE (FR)**
• **MARIE-NELLY, Hervé Dominique**
**75005 PARIS (FR)**
• **COURNAC, Axel**
**75012 PARIS (FR)**

(74) Representative: **Santarelli**
**49, avenue des Champs-Elysées**
**75008 Paris (FR)**

(54)  **Method, device, and computer program for assembling pieces of chromosomes from one or several organisms**

(57)     The invention relates to assembling a sequence representing pieces of at least one chromosome from a set of raw sub-sequences representing DNA fragments of a library including DNA fragments comprising chains of contiguous nucleotides and including DNA fragments comprising combinations of at least two chains of contiguous nucleotides. After having obtained first values representing contact frequencies between DNA regions, the first values being associated with second values representing distances between the corresponding DNA regions, the following steps are iteratively carrying out:
- updating a genome structure on the basis of the first and second values and on the basis of a theoretical model associating a contact probability between DNA regions with a distance between the corresponding DNA regions, the updated genome structure being representative of the real genome structure of the chromosome; and
- updating parameters of the theoretical model as a function of the updated genome structure.

Fig. 9

**Description**

FIELD OF THE INVENTION

The invention generally relates to the field of genome assembly. More particularly, the invention concerns a method, a device, and a computer program for assembling one or multiple genomes of one or multiple organisms using chromosome conformation capture combined with high-throughput sequencing.

BACKGROUND OF THE INVENTION

Microbial communities are fundamental in maintaining environmental stability and healthy living organisms. Microbial species were originally studied individually and have led to the development of numerous technologies in fields as diverse as agronomics, medicine, or depollution. Thanks to advances in technologies, it is now possible to study, through a metagenomic approaches, microbial communities in their complexity (for instance more than 100,000 different species coexist in one gram of soil, or billions of microbes in the human body).

Metagenomic studies mainly consist in collecting, sequencing, and analyzing the genetic material directly extracted from microbial communities directly collected from environments as diverse as skin, abysses, gut, soil, water, etc. Millions of random pieces of DNA (Deoxyribonucleic acid) molecules, which can be cloned into vectors, constitute a library that can cover hundreds of thousands of different species. *In silico* and experimental analysis of such a library lead to the discovery of new genes and enzymes, new networks, and potentially new species (among the more than ten million species still undiscovered on earth).

This new approach is not only currently revolutionizing our understanding of the world, but is about to boost industrial applications most notably in the medicine, energy, and agronomics fields. Many companies have started digging into the enormous resources of this unknown microbial diversity whereas several agencies and academies predict tremendous perspectives regarding the future of this new field. Rapid improvements in sequencing depth, read length, and qualities have led the characterization of genomes of relatively low abundant species within metapopulations.

However, metagenomic analysis of microbial populations remains limited by the difficulty to pool contigs and assemble scaffolds of large chromosomal regions of individual species, impairing the full exploitation of the information contained in their genomes.

Genome sequencing aims at determining the order of nucleotides within DNA molecules. DNA molecules consist of two biopolymer strands coiled around each other to form a double helix. Each strand of this molecule is a polymer of an elementary unit called a nucleotide. Nucleotides are made up of three distinct parts: a cyclic base (made of Guanine - G, Adenine - A, Thymine- T, or Cytosine - C), a cyclic sugar (deoxyribose), and a phosphate group. In a DNA molecule, the nucleotides are joined to one another in a chain by covalent bonds between the sugar of one nucleotide and the phosphate of the next nucleotide, resulting in an alternating sugar-phosphate backbone. According to base pairing rules (A with T and C with G), hydrogen bond the nitrogenous bases of the two separate polynucleotide strands to make double-stranded DNA.

Knowing DNA sequences (i.e. the sequential order of the four cyclic bases) is essential for biological research, and in numerous applied fields such as diagnostic, biotechnology, forensic biology, and biological systematics. Since chromosomes typically comprise several hundreds of millions of pairs of nucleotides, the throughput of DNA sequencers is a key factor for a number of practical uses such as diagnosis.

Massive DNA sequencing methods typically generate sequences (i.e. reads) of few hundreds of base pairs or less. Accordingly, before sequencing a complete genome, it is necessary to shear it into smaller pieces of DNA. These fragments are individually sequenced, at least partially, to determine the corresponding order of nucleotides. Only small portions of those DNA fragments can be sequenced (around 100 to 200 bp). It is to be noted that it is possible to sequence both extremities of DNA fragments using paired end sequencing resulting into two reads. Next, the obtained short sequences have to be re-assembled to provide the overall sequence of the studied genome.

According to the well-known shotgun sequencing method, genomes are extracted from organisms and sheared into small pieces of DNA. Next, the DNA fragments are sequenced and the resulting reads are recombined with each other, based on perfect similarities of overlapping sequences, to form portions of DNA of known sequences that are referred to contigs.

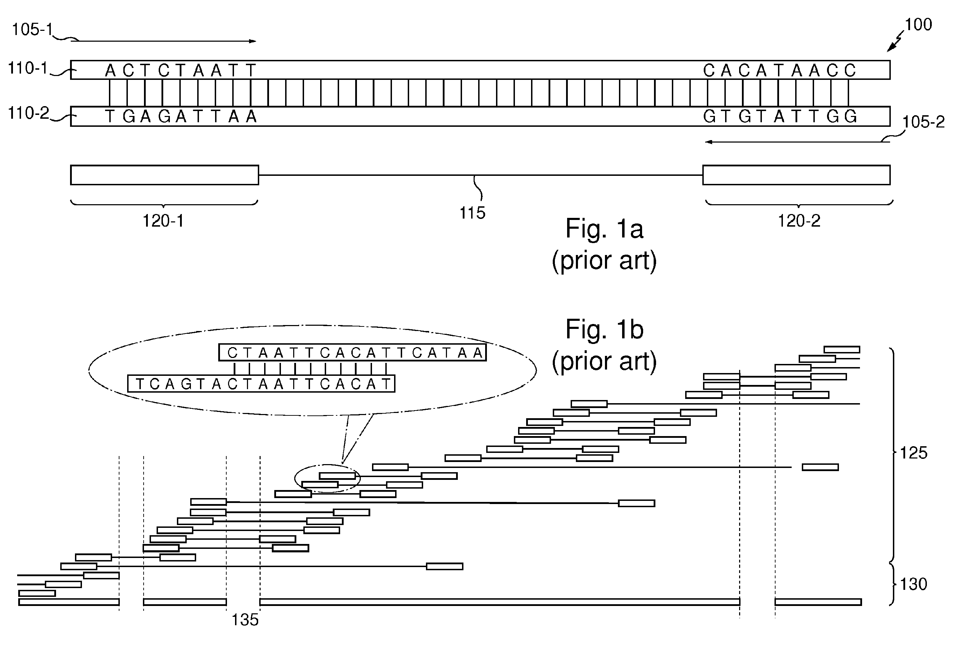

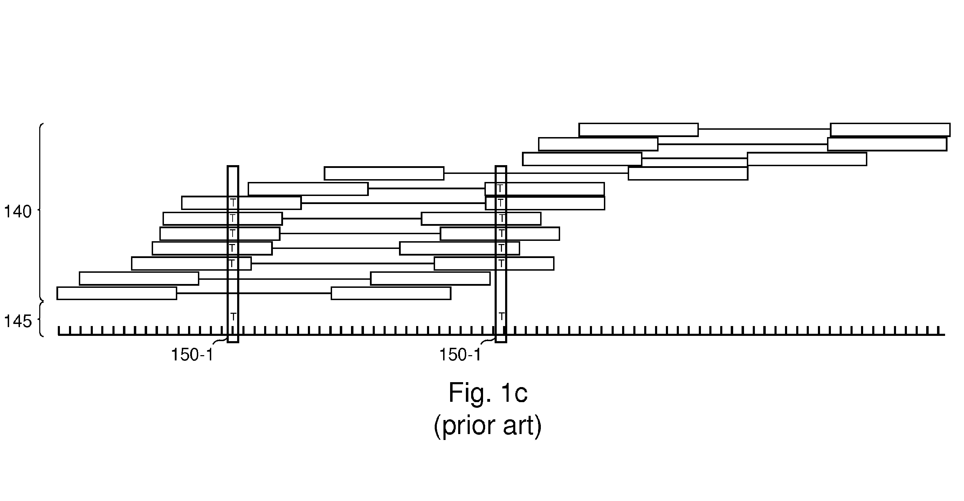

**Figure 1,** comprising Figures 1a, 1b, and 1c, schematically illustrates the process for combining millions of reads in order to form contigs and thus re-assembling fragments.

As illustrated in Figure 1a, a DNA fragment 100 comprises two polymer strands 110-1 and 110-2 including nucleotides that form base pairs (bp), for example the pair of sequences "ACTCTAATT" and "TGAGATTAA". As described above, DNA fragment 100 can only be sequenced inwards from each end (arrows 105-1 and 105-2).

A DNA fragment such as DNA fragment 100 is typically represented with a short line 115 that ends 120-1 and 120-2, representing the sequenced parts of the DNA fragment, i.e. the reads, are thicker.

Figure 1b illustrates the process of assembling DNA fragments 125 into an assembled region 130. To that end, the reads are analyzed and compared to each other to determine the common chains of nucleotides. When two DNA fragments comprise the same chain of nucleotides, they are aligned as a function of the relative position of that chain in the DNA fragments, as illustrated.

Next, aligned DNA fragments can be assembled to form contigs, that is to say pieces of a DNA chromosome. It is to be noted that it may exist parts that do not correspond to any read, forming a gap in the assembled region, for example gap 135.

In other words, a genome is assembled from DNA fragments as a function of overlapping matching sequences of nucleotides.

Figure 1c illustrates a re-sequencing process according to which assembled regions or contigs 140 are re-sequenced by aligning short reads against a reference genome 145, as illustrated with references 150-1 and 150-2.

Such an analysis of reads is generally carried out recursively on a computer to assemble fragments by an assembly algorithm. For the sake of illustration, it can be carried out by the algorithm known under the names of IDBA-UD.

The shotgun sequencing method combined with a program implementing an assembly algorithm can be used to analyze and re-assemble several million reads to obtain contigs that typically comprise up to 30,000 base-pairs.

However, although this method may be efficient, it encompasses limitation due to large repeated portion of the genome introducing ambiguities during the assembly step and leaving the assembly incomplete.

To improve the assembly efficiency, the spatial structure of DNA can be used. Indeed, theoretical knowledge of spatial structure of DNA combined with observed contacts between fragments that result from DNA loops can be used to solve conflicts/ambiguities between contigs or to help close gaps in DNA sequences.

For example, by determining that two fragments are spatially close to each other, one may conclude that these fragments are close to each other along the DNA fiber from which these fragments have been obtained. This mainly results from the polymer physics (chromosomes are semi-flexible polymer chains that frequently loop onto themselves for small genomic separations).

Determining the spatial proximity of two parts of DNA can be done using the chromosome conformation capture (3C) technique.

The 3C technique and its subsequent genomic variants (e.g. 4C, 5C, and Hi-C) are used to analyze the organization of chromosomes.

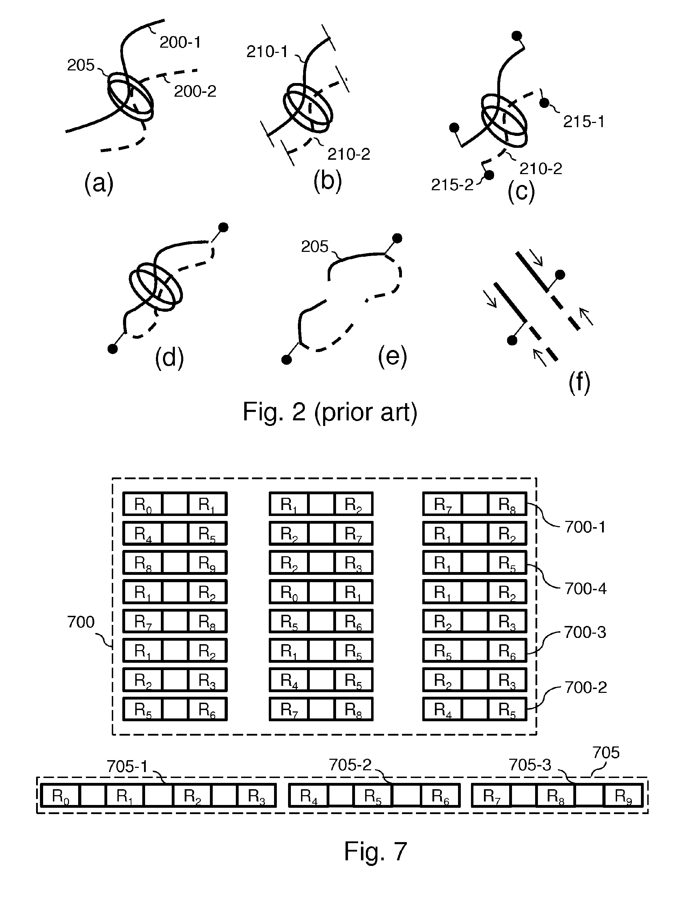

**Figure 2,** comprising Figures 2a to 2f, schematically illustrates main steps of the Hi-C technique.

As illustrated in Figure 2a, a first step is directed to cross-linking parts of DNA that are close together, e.g. parts 200-1 and 200-2. This can be done using formaldehyde for cross-linking parts of DNA to proteins, e.g., proteins 205, and for cross-linking proteins with each other. This leads to cross-linking parts of DNA that are in contact.

Next, in a second step, the cross-linked DNA fragments are fragmented by using a restriction enzyme. Accordingly, as illustrated in Figure 2b, fragments 210-1 and 210-2 result from the fragmentation of parts 200-1 and 200-2, respectively.

A third step aims at filling fragment overhangs with modified bases that generate blunt ends (so as to increase the proportion of chimeric molecules), for example biotin. As illustrated in Figure 2c, the ends of the fragment 210-2 are filled with material 215-1 and 215-2 that generate blunt ends and allow immuprecipitation of the ligated fragments.

In a following step illustrated in Figure 2d, DNA fragment ends are ligated.

Next, cross-linking is reversed and DNA is purified. As illustrated in Figure 2e, the DNA fragments are sheared and fragments that include a litigation junction are isolated.

Finally, as illustrated in Figure 2f, sequencing adaptors are added to DNA molecules to generate a library that can be sequenced. Sequencing these fragments allow identifying the parts of the DNA that are close together due to the spatial structure of the chromosome.

Knowledge of the spatial structure of sequenced pieces of DNA may also be used to identify parts of DNA belonging to different chromosomes of one or several organisms.

Indeed, one may consider that contacts between parts of DNA can be used to cluster parts of DNA belonging to the same sequence (by considering that parts of DNA that are in contact belong to the same sequence).

Accordingly, to carry out a *de novo* sequencing of several chromosomes issued from a single or from several organisms, one may generate a first library using a method of the Hi-C type and a second library using a method of the shotgun type.

Then, contigs generated thanks to the second library (i.e. the shotgun type) may be clusterized as a function of the spatial contact information contained by the first library (i.e. the HiC type) resulting in regrouping contigs into larger group of contigs.

SUMMARY OF THE INVENTION

Faced with these constraints and limitations, the inventors provide a method, a device, and a computer program for high-throughput assembly of pieces of chromosomes from a single organism or from a mix of organisms.

It is a broad object of the invention to remedy the shortcomings of the prior art as described above.

According to a first aspect of the invention there is provided a method for computer for assembling at least one sequence representing at least one piece of at least one chromosome of at least one organism, based on a set of raw sub-sequences representing all DNA fragments of at least one library, the at least one library including DNA fragments comprising chains of contiguous nucleotides of the at least one chromosome and including DNA fragments comprising combinations of at least two chains of contiguous nucleotides of the at least one chromosome, the method comprising the steps of:

- obtaining first values representing contact frequencies between DNA regions of the at least one chromosome, the first values being associated with second values representing distances between the corresponding DNA regions; and
- iteratively carrying out the steps of:

  - updating a genome structure on the basis of the first and second values and on the basis of a theoretical model associating a contact probability between DNA regions with a distance between the corresponding DNA regions, the updated genome structure being representative of the real genome structure of the at least one piece of the at least one chromosome of the at least one organism; and
  - updating parameters of the theoretical model as a function of the updated genome structure.

The claimed method provides an efficient tool to correct and improve genome assembly from species whose genomes are not fully characterized.

In particular, the method of the invention uses the frequent physical contacts experienced by chromosomes sharing similar cellular compartment to assemble large scaffolds of individual chromosomes in species, without prior knowledge to the number of chromosomes expected. Moreover, the method of the invention applies to mixes of cells from different organisms, allowing the deconvolution of metagenomic data. The reads generated when carrying out the method of the invention can also directly be used to generate large DNA contigs by performing a preliminary *de novo* assembly step. Furthermore, the method of the invention enables generating large contigs for the organisms present in the population, and binned together, in one single experiment. It is envisioned that very small metapopulations could be investigated with this approach. Numerous kinds of microbiome can be analyzed with the technique of the invention.

Moreover, the invention provides a high-throughput characterization of the 3D genome structure of known organisms since the invention makes it possible to assess both genome structure (i.e. the sequence) and the 3D genome organization of tens of organisms at a time. Such an approach is of interest to many labs or groups aiming at characterizing for instance chromosomal rearrangements in several organisms, or also the 3D organization of these genomes.

The method of the invention provides a way to characterize the 3D genome structure of unknown species. The metagenomic approach shows that a blind analysis can unveil both genome sequence and the 3D organization of unknown species present in a natural mix of species. Chromosome 3D organization is correlated with metabolic state. Therefore, characterizing the 3D organization of species coexisting in a mix unveils their metabolic state. That could be apply to decipher the growth state of these species compare to each other's, and identify different stages during the evolution of the metapopulation (for instance, stress, rapide growth, quiescence).

In an embodiment, a distance between two DNA regions is determined as a function of a distance between the two DNA regions along a predetermined path and/or a spatial distance between the two DNA regions.

In an embodiment, the method further comprises a step of splitting the raw sub-sequences representing all DNA fragments of at least one library into a plurality of bins.

In an embodiment, the method further comprises a step of generating a plurality of genome candidate structures and of computing an explicit likelihood value for each of the generated candidate genome structures of being closer to the real genome structure.

In an embodiment, the step of generating a plurality of genome candidate structures is based on predetermined structural variations comprising at least one variation among a translocation, a deletion, an inversion, and a duplication.

In an embodiment, one of the generated genome candidate structures is selected as a function of the associated likelihood value according to a rule of the multiple try Metropolis type.

In an embodiment, genome candidate structures are determined by structural variations of bins.

In an embodiment, the step of updating the theoretical model parameters is based on an algorithm of the Gibbs sampler type.

In an embodiment, the theoretical model comprises at least one parameter representative of a threshold used to discriminate intra-chromosomic contacts between DNA regions from intra-chromosomic and inter-chromosomic contacts between DNA regions.

In an embodiment, the theoretical model comprises at least one parameter representative of a threshold used to discriminate intra-chromosomic contacts between DNA regions or intra-chromosomic and inter-chromosomic contacts between DNA regions from contacts between different organisms.

In an embodiment, the method further comprises a step of clustering DNA fragments of the at least one library, each cluster being associated with a specific organism, the raw sub-sequences corresponding to the clustered DNA fragments being processed for sequencing on a cluster basis.

In an embodiment, the step of clustering DNA fragments of the library is based on an algorithm of the Louvain type.

In an embodiment, the method further comprises a step of identifying at least one DNA sequence in the at least one sequence representing the at least one piece of the at least one chromosome of the at least one organism.

In an embodiment, the method characterizes a global chromosome organization of at least one organism, the method further comprises a step of inferring a metabolic state of the at least one organism that global chromosome organization is characterized from the tridimensional organization of the corresponding genome.

A second aspect of the invention provide a method for identifying a genome of an eukaryotic cell, of a prokaryotic cell, or of a microorganism in a biological sample, the method comprising each of the steps of the method described above for assembling at least one piece of at least one chromosome of at least one organism.

In an embodiment, the method is used for identifying a genome of a microorganism in a biological sample, the microorganism being of one of the parasite, bacterium, archaea, fungi, yeast, and virus types.

Those cells and microorganisms can be pathogenic, namely for plants or animals, or non-pathogenic. In a more particular embodiment, the biological sample contains or comprises more than one cell or microorganism species.

In an embodiment, the method for assembling at least one piece of at least one chromosome of at least one organism or the method for identifying a genome further comprises the steps of:

- cross-linking pieces of chromosomes of a prepared biological sample comprising the at least one piece of the at least one chromosome;
- fragmenting the cross-linked chromosomes using restriction enzymes of at least two different types; and
- sequencing the fragments of chromosomes resulting from the fragmenting step.

A third and a fourth aspect of the invention provide an apparatus comprising means configured for carrying out each step of the method described above, and a computer program product for a programmable apparatus, the computer program product comprising instructions for carrying out each step of the method described above when the program is loaded and executed by a programmable apparatus. The claimed apparatus and computer program provides an efficient tool to correct and improve genome assembly from species whose genomes are not fully characterized.

A fifth aspect of the invention provides a method for assembling at least one piece of at least one chromosome of at least one organism, the method comprising the steps of:

- preparing a biological sample comprising the at least one piece of the at least one chromosome;
- cross-linking pieces of chromosomes of the prepared biological sample;
- fragmenting the cross-linked chromosomes using restriction enzymes of at least two different types;
- sequencing the fragments of chromosomes resulting from the fragmenting step; and
- assembling the sequenced fragments of chromosomes.

The claimed method provides an efficient tool to correct and improve genome assembly from species whose genomes are not fully characterized.

Since parts of the present invention can be implemented in software, parts of the present invention can be embodied as computer readable code for provision to a programmable apparatus on any suitable carrier medium. A tangible carrier medium may comprise a storage medium such as a floppy disk, a CD-ROM, a hard disk drive, a magnetic tape device or a solid state memory device and the like. A transient carrier medium may include a signal such as an electrical signal, an electronic signal, an optical signal, an acoustic signal, a magnetic signal or an electromagnetic signal, e.g. a microwave or RF signal.

In an embodiment, the computer code exploits graphic processing units (GPU) that allow parallel processing of large matrix data. Visualization of DNA fragment and the assembly process can be developed using graphic technology to allow portability on web interface.

BRIEF DESCRIPTION OF THE DRAWINGS

Further advantages of the present invention will become apparent upon examination of the drawings and detailed description. It is intended that any additional advantages be incorporated herein.

Embodiments of the invention will now be described, by way of example only, and with reference to the following drawings in which:

Figure 1, comprising Figures 1a, 1b, and 1c, schematically illustrates the process for combining millions of reads in order to form contigs and thus re-assembling fragments;

Figure 2, comprising Figures 2a to 2f, illustrates main steps of the Hi-C technique;

Figure 3 schematically illustrates main steps of a method according to an embodiment of the invention;

Figure 4 illustrates an example of steps for preparing a biological sample;

Figure 5 illustrates an example of the construction of a Meta3C library from a biological sample prepared as described by reference to Figure 4;

Figure 6 illustrates a first example of using a Meta3C library obtained from a mixture of different organisms for determining genome organizations and genome scaffolds;

Figure 7 schematically illustrates a set of raw sub-sequences corresponding to fragments of a Meta3C library and a set of contigs derived from these raw sub-sequences;

Figure 8 illustrates a second example of using a Meta3C library obtained from a mixture of different organisms for determining genome organizations and genome scaffolds;

Figure 9 illustrates steps of the GRAAL algorithm for determining the genome organization and scaffold of one or several of different organisms;

Figure 10, comprising Figure 10a to 10e, illustrates certain steps represented in Figure 9;

Figure 11, comprising Figure 11 a and 11 b, illustrates the relation that can be established between probabilities of contacts between DNA regions and the distances between these DNA regions;

Figure 12, comprising Figure 12a, 12b, and 12c, illustrates an example of a contact array between DNA regions of a biological sample and of the corresponding genome structure, at three different iterations of the GRAAL algorithm ($t = 0$, $t = 501$, and $t = 4,500$); and

Figure 13 is a block diagram illustrating components of a processing device in which embodiments of the invention may be at least partially implemented.

DETAILED DESCRIPTION OF EMBODIMENTS OF THE INVENTION

According to an embodiment of the invention, the frequent physical contacts experienced by chromosomes sharing similar cellular compartment, that can be measured through a metagenomic capture of chromosome conformation (Meta3C) experiment, can be used to assemble larger scaffolds of the genomes present in a metapopulation. Not only can the Meta3C libraries allow the assembly of large DNA regions based on their contact frequencies, but the reads can directly be used to generate them by performing a preliminary *de novo* assembly step.

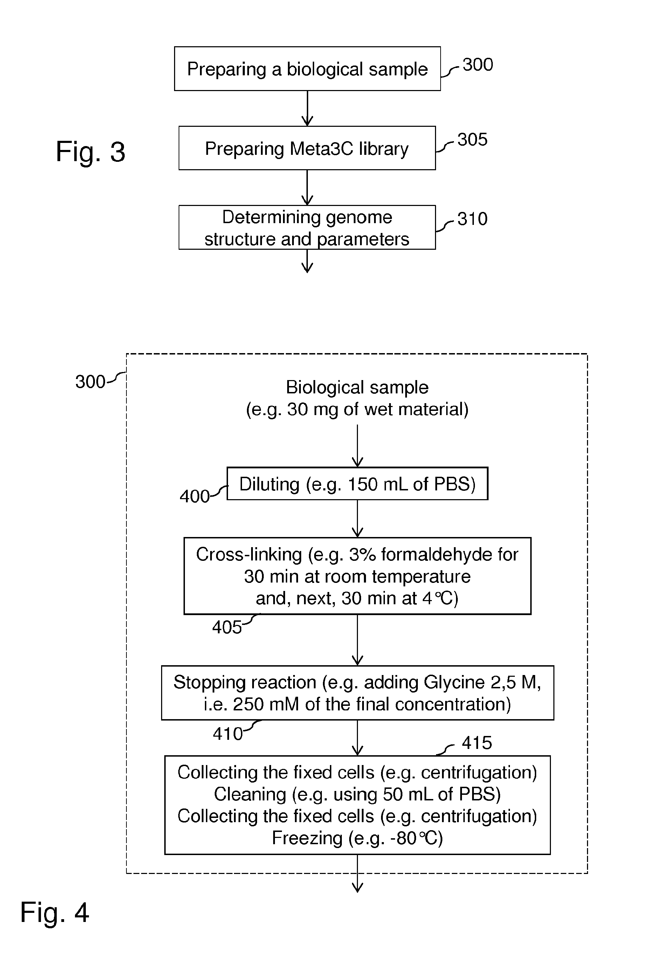

Figure 3 schematically illustrates main steps of a method according to an embodiment of the invention.

As illustrated, a first step (step 300) aims at preparing a biological sample that can be used to build a Meta3C library (step 305). A Meta3C library consists of a set of DNA fragments. The composition of these fragments is determined through pair-end sequencing of their extremities. These sequences, if the protocol does not involve an enrichment step for chimeric fragments, can be used to assemble longer contigs *de novo.* These reads are also used to determine the structure of the genome(s) present within the biological sample, and the associated parameters (step 310).

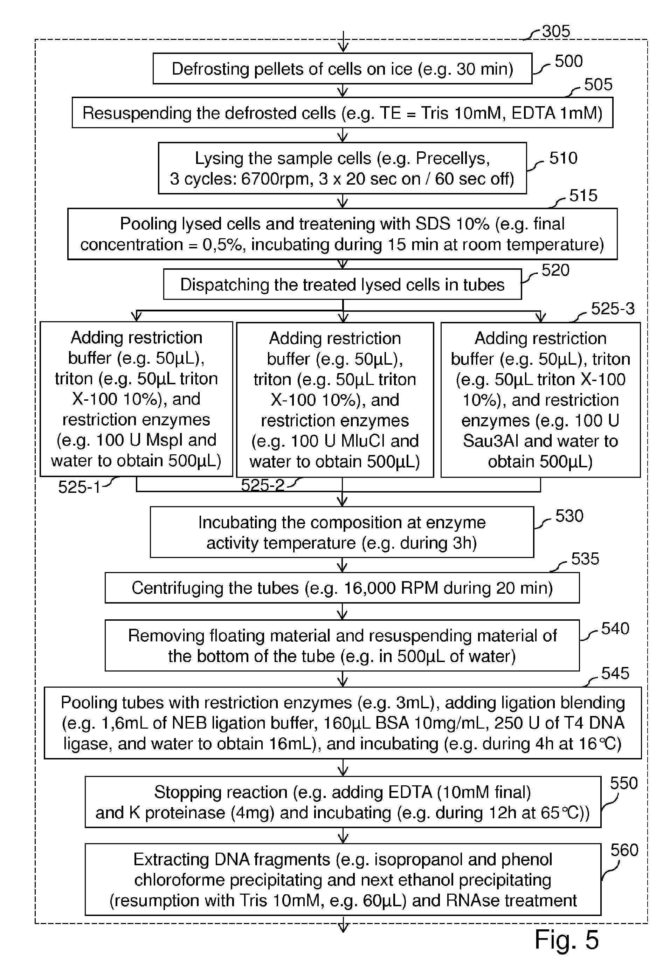

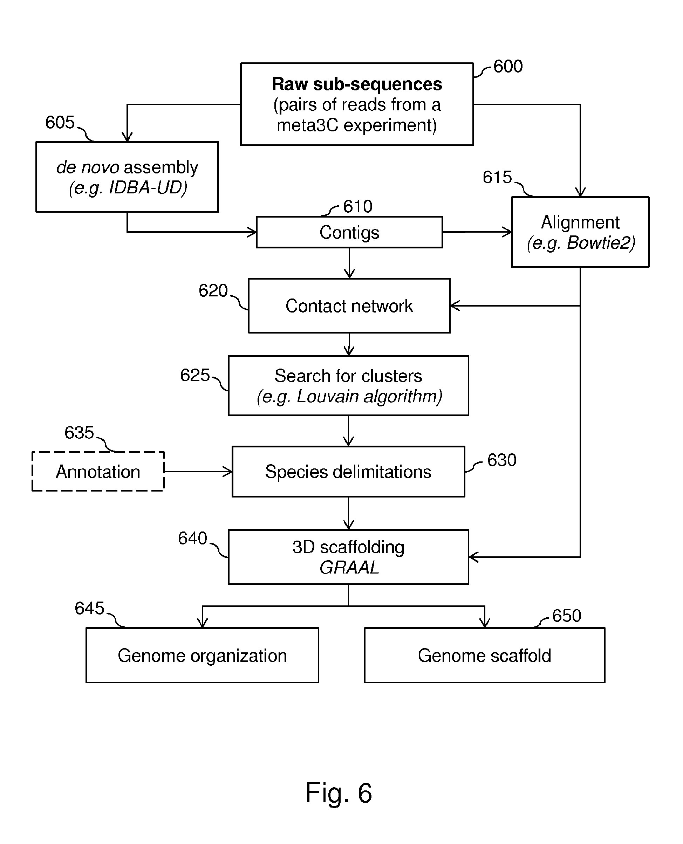

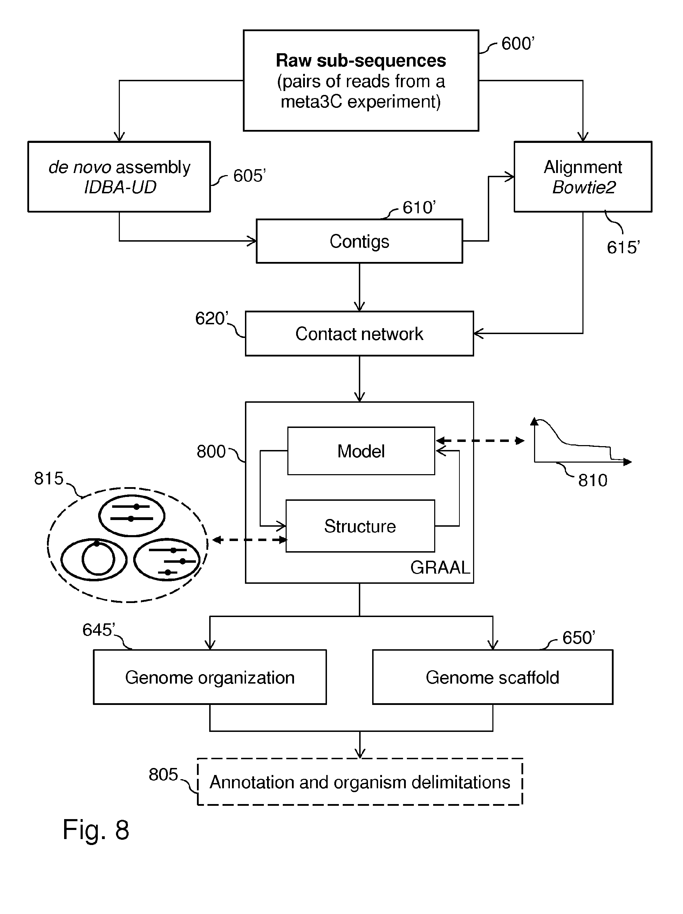

Steps 300 and 305 are described by reference to Figures 4 and 5, respectively, while step 310 is described by reference Figures 6 and 8 representing two examples of embodiments of the invention.

Figure 4 illustrates an example of steps for preparing a biological sample (i.e. step 300 in Figure 3).

A biological sample, for example 30mg of wet material (e.g. wet material issued from river sediments), is diluted (step 400), for example in 150 ml of PBS (Phosphate Buffered Saline). It is to be noted that such a dilution step is not required for solid samples, for samples that need careful manipulation, and/or for samples that are in too small quantities to be handled in large volumes

Next, it is treated chemically in order to freeze the global chromatin network by cross-linking proteins and portions of DNA (step 405). Such a treatment can be obtained with fresh formaldehyde (e.g. 3% final concentration) for 30 minutes at room temperature followed by 30 minutes at 4°C.

In a next step, the cross-linking reaction process is stopped (step 410). For the sake of illustration, the formaldehyde can be quenched with a final concentration of 0.25 M glycine for 5 minutes at room temperature followed by 15 minutes at 4°C.

Next, the fixed cells are collected, typically by centrifugation, washed (for example by using 50 mL of PBS), collected again (e.g. by centrifugation), and frozen in dry ice and stored at -80°C until use (step 415).

One must note that the steps aiming at obtaining an initial biological sample with a consistent cross-link of DNA fragments (step 405) are essential. Therefore, a peculiar care is to be brought to the generation of the fixed cells to obtain a sufficient amount of cross-linked fragments.

Figure 5 illustrates an example of the construction of a Meta3C library (step 305 in Figure 3) from a biological sample prepared as described above.

As illustrated, a first step (step 500) aims at defrosting frozen pellets of cells. For the sake of illustration, this can be done on ice over 30 minutes. Next, the defrosted cells are resuspended (step 505). This can be done in a final volume of 650 $\mu$l 1X TE pH 8.

In a following step, the sample cells are lysed (step 510). This can be done in a device known under the Precellys trademark. For the sake of illustration, the sample cells can be lysed over three cycles of 20 seconds that are carried out every 60 seconds with a speed of 6,700 rotations per second.

Next, the lysed cells are pooled and treated with SDS (Sodium Dodecyl Sulfate) at a concentration of 10% cc to obtain, for example, a final SDS concentration of 0.5% cc, and the pooled cells are incubated, for example for 15 minutes at room temperature (step 515)

The treated lysed cells are then dispatched in several tubes (step 520). The DNA molecules present within these cells are then fragmented with different restriction enzymes (steps 525-1 to 525-3, generically referred to as 525).

Since the original biological sample typically comprises cells of different organisms, several types of restriction enzymes can be used so that different types of chromosome composition (regarding the proportion of GC and AT base pairs) can be fragmented into pieces whose mean size does not exceed a predetermined threshold (typically 500 to 1,000 base-pairs). This threshold is particularly important for Meta3C libraries that do not present enrichment steps for chimeric reads.

It is to be noted that, although only three different restriction enzymes are represented in Figure 5 (steps 525-1 to 525-3), any number of restriction enzymes can be used, either in combination or in independent processes.

As illustrated in step 525, the lysed cells are put in tubes containing a digestion mix (e.g. 1X NEB 1 buffer (10mM Bis-Tris-Propane-HCl, 10mM $MgCl_2$, 1mM DTT, ph 7.0 at 25°C, New England Biolabs), 1% Triton X-100, and 100U restrictive enzyme of a predetermined type). The blend of lysed cells and digestion mix is incubated (step 530), at the corresponding enzyme activity temperature, for example 37°C, for a predetermined period of time, for example three hours.

According to a particular embodiment, the restriction enzymes that are used are chosen so as to recognize four base-pair combinations. Accordingly, the cross-linking step that is carried out for preparing a biological sample requires a concentration and a period of time that are greater than those generally used for preparing a DNA fragment library. Enzymes recognizing a six base-pair combination or others can be used, but the construction of the library requires an enrichment step, for instance incorporation of biotinylated bases, at the edges of the DNA fragments.

Next, in a following step, the tubes containing the blend of lysed cells and digestion mix are centrifuged (step 535), for example at a rotation speed of 16,000 rotations per minute for 20 minutes.

After centrifugation, the floating material (supernatant) is removed and the material deposited on the bottom of the tubes is resuspended (step 540), for example in 500$\mu$l of water.

The tubes are then pooled (e.g. 3 mL) and a ligation mix is added to the obtained mix (step 545). For the sake of illustration, the ligation mix can be based on a NEB (New England Biolabs) ligation buffer (e.g. 1.6 mL), BSA (Bovine Serum Albumin) (e.g. 10mg/mL, 160$\mu$l), 250 U of T4 DNA ligase, and water to obtain a volume of 16mL. The mix is incubated, for example for four hours at 16°C.

Next, the reaction is stopped and the cross-link is reversed (de-cross-linking step 550). This can be done by addition of EDTA (Ethylenediaminetetraacetic acid) to the mix, for example 100mM final, and K proteinase, for example 4mg, and by incubating the mix, for example at 65°C for 12 hours.

The DNA fragments are then extracted (step 560), for example by precipitating isopropanol, precipitating phenol chloroform, and by precipitating ethanol (e.g. resumption with Tris 10mM, e.g. 60$\mu$L), and a RNAse treatment is applied to get rid of RNA molecules.

Finally, all tubes are pooled, resulting in a Meta3C library that is quantified on gel using, for example, the application known as Quantity One that is developed by Bio-Rad (Quantity One and Bio-Rad are trademarks).

The Meta3C library obtained by carrying out the steps described by reference to Figures 4 and 5 typically comprises reads of a few hundreds of base pairs or less. Overlapping reads of the library can be pieced together into larger contiguous sequences (contigs) by using standard assembly algorithms such as IDBA-UD.

It is to be noted that the Meta3C library obtained by carrying out the steps described by reference to Figures 4 and 5, without an enrichment step for chimeric sequences, mainly comprises DNA molecules corresponding to fragments of chromosomes present in the biological sample (i.e. chains of contiguous nucleotides of a chromosome) from which is derived library is derived. These fragments are called DNA fragments of the shotgun type since they are similar to DNA fragments recovered in shotgun sequencing libraries. These shotgun DNA fragments represent about 80% of the fragments of the library.

The Meta3C library also comprises DNA molecules, also called chimeric molecules, that are the combinations of two sequences trapped together during the crosslinking step (i.e. combinations of at least two different chains of contiguous nucleotides). The more frequently these two sequences are close to each other (step 405 of Figure 4), the more frequently they will be trapped together. These sequences can be positioned far apart along the same DNA fiber (chromosome) or onto separate chromosomes (i.e. a chimeric molecule is a combination of at least two different chains of contiguous nucleotides of the same chromosome or of at least two different chromosomes).

These long distance interactions between DNA fragments represent about 20% of the molecules of the library. Contrary to the chimeric molecules present in a Hi-C library, where an enrichment step for these chimeric molecules is performed, the chimeric molecules in a Meta3C library do not comprise biotin between the combined sequences.

Accordingly, a Meta3C library comprises DNA molecules that are both of the "shotgun" and "chimeric" forms, and thus, can be used to generate contigs and to scaffold them in a single experiment.

It is to be noted that a MetaHiC library, where the "shotgun" molecules are in lesser quantities, can be used to scaffold contigs obtained through other means.

**Figure 6** illustrates a first example of using a Meta3C library obtained from a mixture of different organisms for determining genome organizations and genome scaffolds.

The algorithm illustrated in Figure 6 is based on a set of raw sub-sequences referenced 600 that is obtained from a Meta3C library. Raw sub-sequences are pairs of reads resulting from the pair-end sequencing of DNA fragments present in the Meta3C library.

A first step aims at assembling DNA fragments of the Meta3C library into larger sequences called contigs (step 605). This step can be carried out by a standard assembly algorithm such as IDBA-UD. As a result, a set of contigs, referenced 610, is obtained.

The paired-ends information of the reads constituting the different contigs is then used to generate contact network of all contigs against each other. To that end, the pair-end reads obtained from the sequencing of the Meta3C library are aligned on the contigs 610 obtained as a result of step 605 using a read alignment application (step 615).

It is to be recalled that a read alignment application, also called an aligner, makes it possible to align (i.e. to position) reads along a larger sequence of DNA such as a chromosome (or a portion of chromosome) that is used as a reference. For the sake of illustration, the application known as Bowtie 2 is an aligner and can be used to carry out step 615).

The pair-end information contained in the DNA molecules determines a contact network between the contigs, referenced 620, by revealing which contigs are in contact with which other contigs. The frequency with which these interactions between contigs are found unveils the strength of the links bounding them.

For example, turning to Figure 7, one can determine a contact network of reads.

**Figure 7** schematically illustrates a set 700 of raw sub-sequences corresponding to fragments of a Meta3C library and a set 705 of contigs derived from these raw sub-sequences (e.g. according to steps 605 and 615 described by reference to Figure 6). As illustrated, each of the raw sub-sequence ends represent sequenced data (these sequenced data being the reads). The raw sub-sequences are combined when they share the same read to form a contig.

For the sake of illustration, only twenty four raw sub-sequences corresponding to twenty four DNA fragments are represented, each sub-sequence comprising a paired-end of reads. For example, sub-sequence 700-1 comprises paired-end reads $R_7$ and $R_8$.

Similarly, only three contigs are represented (705-1, 705-2, and 705-3). As illustrated, each contig is based on a sub-set of sub-sequences and comprises a paired-end of reads as well as internal reads (that have been used for assembly purposes). For example, contig 705-1 comprises paired-end reads $R_0$ and $R_3$.

As described above, the use of a standard assembly algorithm such as IDBA-UD enables assembly of DNA fragments by comparing the reads to form contigs. Accordingly, sub-sequence 700-2 can be combined with sub-sequence 700-3 to form contig 705-2 (i.e. contig $R_4$-$R_5$-$R_6$).

For the sake of illustration, it can be assumed that contigs 705-1 and 705-2 belong to the same chromosome while contig 705-3 belongs to a different chromosome. In this case, the link between $R_3$ and $R_4$ is weak, impairing the assembly of these fragments.

Construction of a contact network reveals items of information contained in chimeric molecules that are made of distant, non-contiguous sequences. For example, in view of the distance between reads $R_1$ and $R_5$ in the contigs 705-1 and 705-2, sub-sequence 700-4 is deemed to be associated to a chimeric molecule.

As illustrated in Figure 7, the links that can be established from the set 705 of sub-sequences follows a contiguous structure until positions $R_4$/$R_5$ and $R_6$/$R_7$. At these positions, gaps occur since, for some reasons, no pair-end information has been found to link the contigs.

However, long distance items of information can be identified from the pair-end items of information of chimeric molecules that were not used in the assembly. Such items of information reveal connections between contigs that are not apparent from the assembly process. Long distance items of information can be used to position contigs aside one another in their most likely orientation.

A contact array can be constructed from the set 700 of sub-sequences and from the obtained set 705 of contigs, by determining the position of each sub-sequence end in the contigs (such a distance can be expressed as a function of a number of base pairs or as a function of a number of restriction fragments) and by associating a number of contacts (or a contact frequency) with each of these positions.

To that end, all the contigs are assembled in a linear structure, the sequence of reads of the contigs forming the references of the lines and of the columns of the contact array as illustrated herein below:

|       | $R_0$ | $R_1$ | $R_2$ | $R_3$ | $R_4$ | $R_5$ | $R_6$ | $R_7$ | $R_8$ | $R_9$ |
|-------|-------|-------|-------|-------|-------|-------|-------|-------|-------|-------|
| $R_0$ | 0     | 2     | 0     | 0     | 0     | 0     | 0     | 0     | 0     | 0     |
| $R_1$ | 2     | 0     | 5     | 0     | 0     | 2     | 0     | 0     | 0     | 0     |
| $R_2$ | 0     | 5     | 0     | 4     | 0     | 0     | 0     | 1     | 0     | 0     |
| $R_3$ | 0     | 0     | 4     | 0     | 0     | 0     | 0     | 0     | 0     | 0     |
| $R_4$ | 0     | 0     | 0     | 0     | 0     | 3     | 0     | 0     | 0     | 0     |
| $R_5$ | 0     | 2     | 0     | 0     | 3     | 0     | 3     | 0     | 0     | 0     |
| $R_6$ | 0     | 0     | 0     | 0     | 0     | 3     | 0     | 0     | 0     | 0     |
| $R_7$ | 0     | 0     | 1     | 0     | 0     | 0     | 0     | 0     | 3     | 0     |
| $R_8$ | 0     | 0     | 0     | 0     | 0     | 0     | 0     | 3     | 0     | 1     |
| $R_9$ | 0     | 0     | 0     | 0     | 0     | 0     | 0     | 0     | 1     | 0     |

To fulfill the contact array, each cell of the latter is, in an initialization step, set to zero. Next, the sub-sequences of the set of sub-sequences are selected (typically one after another) and, for each sub-sequence, the position of the read corresponding to one end of the sub-sequence is determined in the linear structure of assembled contigs to determine a first coordinate ($a$). Similarly, the position of the read corresponding to the other end of the sub-sequence is determined in the linear structure of assembled contigs to determine a second coordinate ($b$). Then, the first and second coordinates are used to identify two symmetrical cells (($a$, $b$) and ($b$, $a$)) of the contact array that content is increased by one. Alternately, only half of the contact array can be fulfilled the other half being duplicated from the first one.

Turning back to Figure 6, a following step is directed to clustering the DNA fragments of the Meta3C library (step 625). This can be done, for example, with an algorithm of the Louvain type.

It is to be recalled that the Louvain algorithm is a simple and efficient method for identifying clusters or communities in complex networks. It is based on the general definition according to which pairs of nodes are more likely to be connected if they are both members of the same community(ies), and less likely to be connected if they do not share any community. Therefore, by knowing the links and the number of links between nodes, one can identify clusters representing communities.

Applying the Louvain algorithm on the entire set of contigs using their overall contact network to cluster them into subsets of contigs exhibiting preferential contact frequencies between them allows the DNA fragments to be clustered according to the organisms to which they belong, as illustrated with reference 630. Therefore, the clusters determined at step 625 are used to classify most of the DNA fragments of the Meta3C library into pools of contigs that can then be processed individually to determine with more precision the scaffold of each of these organisms, as well as (eventually) their genome organization.

As suggested with reference 635, items of information can be associated with each determined cluster.

Next, in a following step, a 3D scaffold algorithm, also called GRAAL (for Genome (Re)-Assembly Assessing Likelihood from 3D contact data), is carried out (step 640) to determine the genome organization and scaffold of each of the organisms, as illustrated with references 645 and 650, respectively.

GRAAL is an algorithm that iteratively applies virtual rearrangements (or structural variations) to an initial set of DNA fragments. The method is based on a probabilistic formulation that calculates the likelihood of a proposed genome structure based on the contact network data and prior (data-independent) assumptions relating expected contact frequencies to genome structures.

These assumptions take advantage of the fact that predicted and observed intrachromosomal contact frequencies are strongly related to the genomic separation between loci, typically following an approximate power law relation and exhibiting a plateau for large genomic separations, where the frequency becomes comparable to inter-chromosomal contact frequencies.

The sub-sequences used for the initialization of the GRAAL algorithm are generated from a set of contigs or from a reference genome. They are advantageously split into regular bins of restriction fragments as small as one restriction fragment.

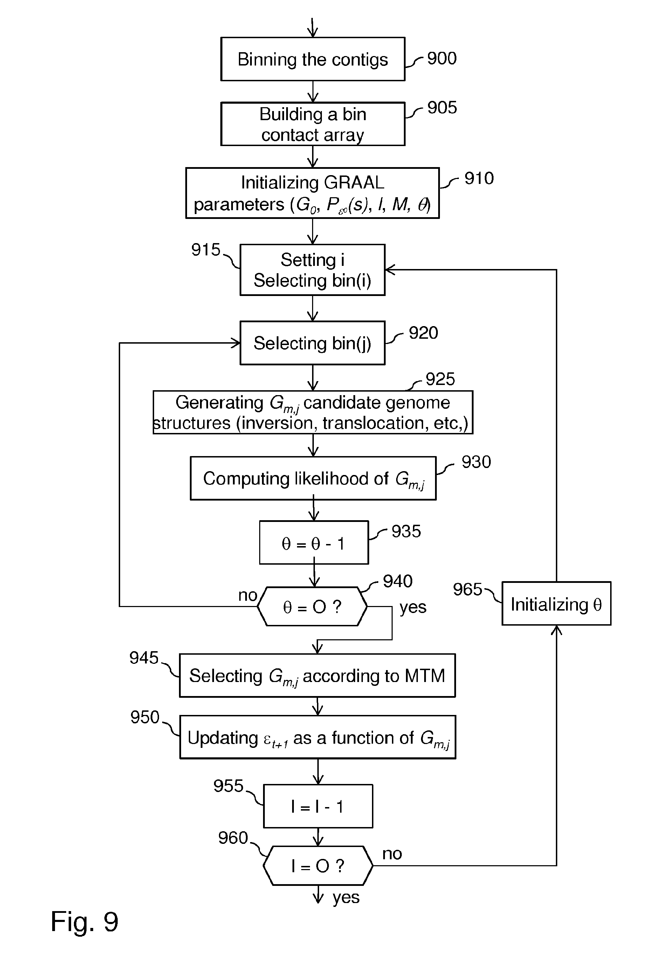

As described by reference to Figures 9 to 11, at each iteration, the GRAAL algorithm picks a new genomic bin and scans the entire genome for $N$ bins, sampled from the measured contact frequencies. Then, considering $V$ different virtual structural variations (including translocations, deletion, inversion, and duplications), a set of genomes relative to these potential neighbors is computed. The candidate bin is then sampled on the local likelihood landscape of this set of genomes, and one of the most likely structures is retained for the next iteration. The position of every bin in the genome, and not only of pre-assembled contigs, is tested independently by the GRAAL algorithm several times, allowing mitigation of assembly errors and identification of very small structural variations.

**Figure 8** illustrates a second example of using a Meta3C library obtained from a mixture of different organisms for determining genome organizations and genome scaffolds in a single analysis step, using GRAAL.

As illustrated, steps 600' to 620' are similar to steps 600 to 620 described by reference to Figure 6, respectively. These steps aim at providing a set of contigs and contact network information.

In a following step (step 800), the obtained set of contigs and the contact network items of information are used to determine directly (i.e., without carrying out a clustering step such as the Louvain algorithm described by reference to step 625 in Figure 6) the genome organization and scaffold of each of the different organisms present in the biological sample used to create the processed Meta3C library, referenced 645' and 650', respectively.

As suggested in Figure 8, the genome organization and scaffold can then be used to characterize the organisms present in the biological sample used to create the processed Meta3C library (step 805).

The step 800 of determining the genome organization and scaffold is preferably based on the GRAAL algorithm briefly introduced by reference to Figure 6 and described in more detail by reference to Figures 9 to 11, wherein a theoretical model of the structure of a genome is adapted to discriminate the chromosomes of different organisms present in the biological sample that is analyzed.

As illustrated, the GRAAL algorithm uses a theoretical model 810 for iteratively determining the structure 815 of a set of chromosomes, this structure being used at each iteration for updating the theoretical model 810.

It is to be noted here that even if not any theoretical model and chromosome structure are illustrated in Figure 6, the GRAAL algorithm carried out at step 640 is also based on the use of a theoretical model and a chromosome structure that interact to converge to the solution.

The GRAAL algorithm is an algorithm of the Monte Carlo Markov Chain (MCMC) type. It aims at exploring the distribution of genomic structure that may be at the origin of the observed data (i.e. Meta3C library). Contact frequencies between elements of a genomic structure, belonging to the same organisms or not, are modeled in a computerized theoretical model. Analyzing the distribution of genomic structures enable determining the probable number of different organisms present in the biological sample used to generate the processed Meta3C library and determining the genome of each of the organisms.

Determining the probable number of different organisms present in the biological sample is based on the frequency contact variation.

For a given Meta3C library, denoted $D$, GRAAL algorithm seeks to estimate the entire probability distribution $p(G|D)$ of one-dimensional genome structure(s) $G$ consistent with the data. The algorithm is based on a probabilistic approach using Bayes' rule $p(G|D) \propto p(D|G)p(G)$. Assuming that in the absence of data, all structures $G$ have equal probability (flat prior), Bayes' rule can be reduced as follows: $p(G|D) \propto p(D|G)$.

The calculation of $p(D|G)$ requires a model to quantitatively predict intrachromosomal contacts and inter-chromosomal contacts that are referred to as *cis*- and *trans* chromosomal contact matrices $M$ for a given $G$.

It is assumed that the *cis* contact probabilities $P_c$ depend on genomic separation $s$ as a power-law followed by a plateau: $P_c(s) = P_t s^b s_0^{-b}$ for $s \leq s_0$ and $P_c(s) = P_t$ for $s \geq s_0$, in accordance with the theoretically predicted and measured behavior of chromosomes confined in a nucleus. It is to be noted that different values of $b$ and $s_0$ have been reported for different organisms or chromosomes.

It is also assumed that *trans* contacts occur with uniform probability $P_t$ per unit genomic length squared, considering the absence of prior information about chromosome neighborhoods and the relative weakness of *trans* contact frequencies relative to *cis* contacts.

The parameters $\varepsilon = (b, s_0, P_t)$, also called nuisance parameters, are estimated by the GRAAL algorithm.

Moreover, it is assumed that counts of the measured contact matrix M obey a Poisson distribution, i.e. that $P(M_{i,j} = k) = \lambda_{i,j}{}^k e^{-\lambda_{i,j}} / k! \ (k \in N)$, $(k \in N)$, where the contact probability $\lambda_{i,j}$ for bin $(i,j)$ is given by $P_t$ or $P_c$ for *trans* or *cis* contacts, respectively.

Together, these assumptions specify a probabilistic model $p(G, \varepsilon|D)$ that allows to calculate the likelihood $p(D|G,\varepsilon)$ of any genome structure $G$ given a Meta3C library (data set $D$).

The Monte Carlo Markov Chain algorithm (i.e. a Gibbs sampler) is used to sequentially generate nuisance parameters and genome structures. The nuisance parameters are updated iteratively, alternately with the changes of the genome structure, by a classic Metropolis algorithm.

A Multiple Try Metropolis sampler, which generates an ordered sequence of genome structures $G_t$, $t = 1,2....$ $N_t$ starting from an initial genome structure $G_0$ is used to generate the genomic structure.

Given a current genome structure $G_t$, a random set of N new structures is computed by applying virtual structural variations which may consist for example, in an insertion, deletion, duplication, inversion, translocation or a simultaneous combination of these. For each candidate structural variation, the likelihood of the new structure is computed as above, and a local stochastic optimization is performed on the space of genome structures to determine the next structure $G_{t+1}$.

The new genome is accepted or rejected with a probability specified by the Metropolis Try Metropolis rule. As opposed to a uniform choice of structural variations, this procedure allows for computationally efficient sampling of the structure probability density.

Finally, after discarding a burn-in period, the Markov chain samples are used to estimate the joint probability distribution $(G, b, s_0)$.

**Figure 9** illustrates steps of the GRAAL algorithm for determining the genome organization and scaffold of one or several of different organisms.

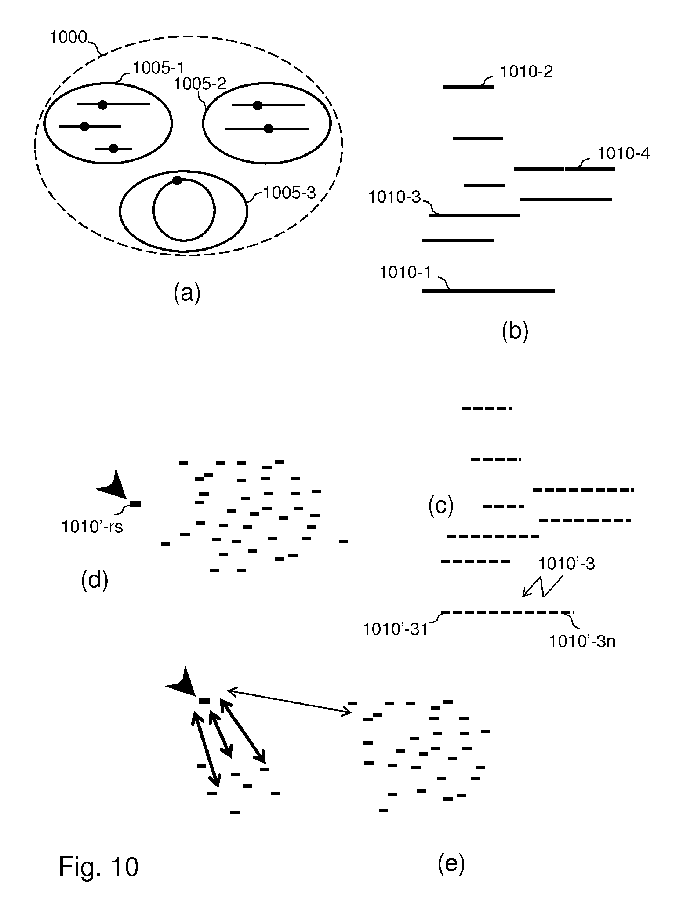

Figure 9 is described in conjunction with **Figure 10,** comprising Figures 10a to 10e, that illustrates certain steps represented in Figure 9.

As illustrated in Figure 10a, a mix 1000 of cells from different organisms, for example cells 1005-1, 1005-2, and 1005-3, comprising a genome associated to the corresponding one of the organisms, is used to create a Meta3c library that sub-sequences representing DNA fragments can be combined to form a set of contigs, such as contigs 1010-1 to 1010-4 represented in Figure 10b.

For the sake of illustration, contig 1010-1 represent an assembly of DNA fragments of cell 1005-1, contig 1010-2 represent an assembly of DNA fragments of cell 1005-2, contig 1010-3 represent an assembly of DNA fragments of cell 1005-3, and contig 1010-4 represent an assembly of DNA fragments of cells 1005-1 and 1005-3. In other words, contig 1010-4 results from a contact between a DNA portion of cell 1005-1 and a DNA portion of cell 1005-3. Contig 1010-4, that does not exist as such in a DNA portion, is also called a chimeric pair of reads.

Turning back to Figure 9, a first represented step is directed to binning the contigs that have been previously determined by a standard assembly algorithm (step 900). According to step 900, each contig is split into bins that represent portions of contig. The length of the bins can be adjusted by a user. It is advantageously determined as a function of the number of contacts between reads and/or as a function of contact frequencies between reads.

According to a particular embodiment, the length of the bins is approximately the same for all the bins and its minimum size is set equal to a restriction fragment (i.e. a DNA fragment resulting from a fragmentation step of a restriction enzyme). The set of $n$ bins obtained after carrying out step 900, corresponding to the whole set of DNA fragment of the Meta3C library, can be expressed under the following relation: $B = \{b_1, b_2, ..., b_i, ..., b_n\}$.

Examples of bins are represented in Figure 10c. For the sake of illustration, contig 1010-3 is split into a set of bins 1010'-3 comprising bins 1010'-31 to 1010'-3n.

Next, turning back to Figure 9, a bin contact array is built (step 905). It is built as a function of the contact network, that is to say as a function of the contact frequencies between reads of contigs. While the contact network defines contacts between a position a of a contig $i$ and a position $b$ of a contig $j$, the bin contact array represents the number of contacts between a bin $a$ of a contig $i$ and a bin $b$ of a contig $j$.

As described above, about 80% of these contacts results from DNA fragments that are adjacent along a DNA fiber and about 20% of these contacts result from DNA fragments that are not are adjacent along a DNA fiber but that are spatially close to each other during the cross-linking step (when building the Meta3C library).

In a following step, parameters of the GRAAL algorithm are initialized (step 910). Among these parameters, the genomic structure $G_t$ that is modified during the execution of the GRAAL algorithm is initialized to reference structure $G_0$ (i.e. $t = 0$). Such a reference structure can be, for example, a genome already assembled, a genome partially assembled, or a set of contigs or of bins. For the sake of illustration, the initial structure of the genomes is set as being the set of contigs that is to say a set of assembled DNA fragments comprising chimeric pairs of reads.

Another parameter of the GRAAL algorithm to be initialized represents the parameters $\varepsilon = (b, s_0, P_t)$ of the theoretical model $P(s)$ of the structure of genomes, the theoretical model being denoted $P_{\varepsilon 0}(s)$ during the initialization step. According to a particular embodiment, the model $P(s)$ associates a contact probability between two bins with the distance between these two bins (the distance being typically determined as a function of the distance between these two bins along a DNA fiber and/or as a function of a spatial distance (e.g. distance equals to one if there is a contact between the two bins and zero otherwise)). Parameters $(b, s_0, P_t)$ can be considered as representing a scale factor and two thresholds $R_0$ and $R_1$ characterizing the links between two bins of the same chromosome, two bins of two chromosomes of one of different organisms, and two bins of two chromosomes of two different organisms. An example of such a theoretical model is illustrated in Figure 10b.

Still another parameter to be initialized, denoted $I$, represents the number of iterations or number of cycles the GRAAL algorithm must run. It represents the number of mutations that the genomic structure is subjected to, that is to say the number of structural variations that can be brought to the genomic structure. For the sake of example, the parameter $I$ can be set to 4,500.

Still another parameter to be initialized, denoted $V$, represents the types of structural variations that the genomic structure is subjected to. Such structural variations include, for example, translocations, deletion, inversion, and duplications of bins, and/or combination of these structural variations.

Still another parameter to be initialized, denoted $\theta$, represents the number of neighboring bins that have to be considered for a given bin when generating candidate genome structures. For the sake of example, the parameter $\theta$ can be set to 10.

After having initialized these parameters, an index $i$ is set to a value comprised between one and the number $n$ of bins of the set $B = \{b_1, b_2, ..., b_i, ..., b_n\}$ and bin $i$ is selected (step 915). The value of index $i$ is chosen so that each bin is selected at least once and preferably the same number of times, e.g. ten.

Figure 10d illustrates an example of a set of bins among which one bin (bin $i$), referenced 1010'-rs, is selected.

Turning back to Figure 9, after having selected bin $i$, a set of $\theta$ bins is selected among the $n$ bins resulting from the binning step 900 (step 920). According to a particular embodiment, the $\theta$ selected bins are chosen as being $\theta$ probable neighbors of bin $i$. This can be done be randomly selecting $\theta$ bins that are associated with high numbers of contacts with bin $i$.

A first of the $\theta$ selected bins, denoted bin(j), is identified to be processed.

Next, selected bin $i$ and identified bin $j$ are then used to generate a set of candidate genome structures denoted $G_{m,j}$ (step 925). These candidate genome structures are determined as a function of bins $i$ and $j$ and of the predetermined types $V$ of structural variations that the genomic structure is subjected to.

For each of the generated genome structures denoted $G_{m,j}$, a likelihood value that the corresponding genome structure is the real genome structure is computed (step 930). A likelihood value is typically obtained by comparing the predicted contacts between bins with the observed data for that structure given the current model parameter $\varepsilon_t$.

After having generated candidate genome structures for identified bin $j$ and computed a likelihood value for each of these candidate genome structures, the parameters $\theta$ is decremented by one (step 935) and a test is carried out to determine whether or not its value is equal to zero (step 940).

If the value of variable $\theta$ is different from zero, the last four steps (steps 920 to 935) are repeated to identify a different bin $j$ of the set of $\theta$ selected bins, to generate a new set of candidate genome structures as a function of the new identified bin $j$, and to compute a likelihood value for each of the generated candidate genome structures.

On the contrary, if the value of variable $\theta$ is equal to zero, that is to say after having generated candidate genome structures for selected bin $i$ and for each of the $\theta$ selected bins, one of the generated candidate genome structures is selected as being the next genome structure $G_{t+1}$ (step 945), i.e. $G_{t+1} = G_{m,j}$. According to a particular embodiment, the selection of one of the generated candidate genome structures is based on the multiple try Metropolis rule (MTM). It is to be noted that other criteria can be used, in particular less restrictive criteria (e.g. a basic stochastic optimization) for optimizing computing performance.

Figure 10e illustrates the step of generating candidate genome structures for selected bin $i$ and for each of the $\theta$ selected bins.

Next, turning back to Figure 9, the parameters $\varepsilon_{t+1}$ ($b$, $s_0$, $P_t$) of the theoretical model $P(s)$ of the structure of genomes are updated in view of the observed data and of the genome structure $G_{t+1}$ (step 950). For the sake of illustration, this can be done by applying a classic Gibbs sampler algorithm.

After having updated the values of the parameters of the theoretical model $P(s)$ of the structure of genomes, variable / is decremented by one (step 955) and a test is carried out to determine whether or not its value is equal to zero (step 960).

If the value of variable / is different from zero, the value of $\theta$ is initialized to the value representing the number of neighboring bins that have to be considered (step 965) and the algorithm is branched to step 915 for selecting a new bin $i$ and then repeating steps 920 to 950 with new selected bin $i$. As mentioned above, each bin of the set of bins resulting from 900 are preferably selected once and advantageously about ten times.

On the contrary, if the value of variable / is equal to zero, the algorithm stops.

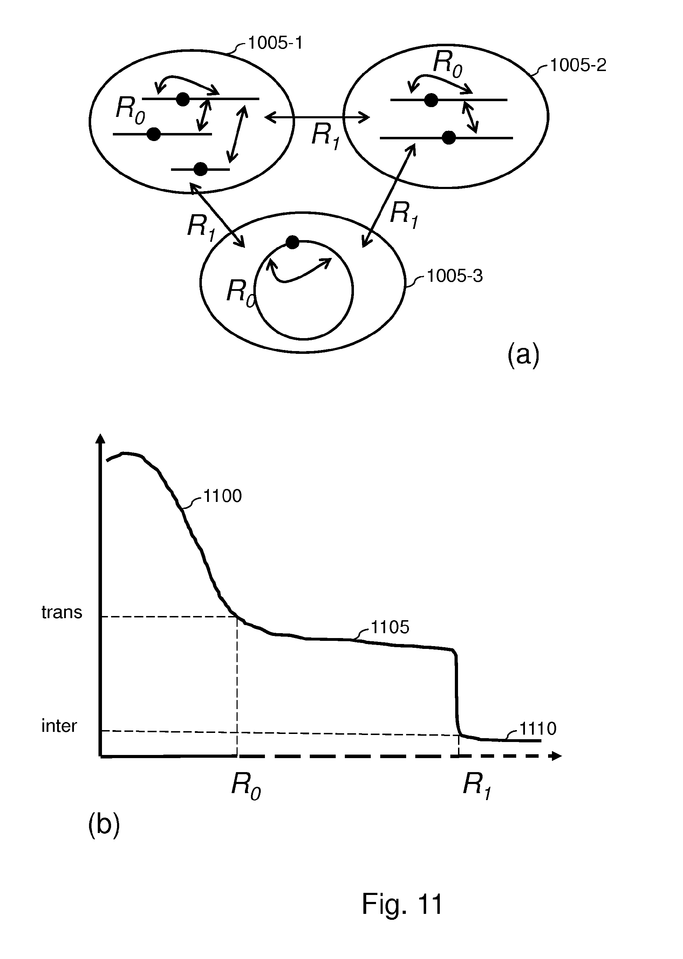

**Figure 11,** comprising Figure 11 a and 11 b, illustrates the relation that can be established between probabilities of contacts between DNA regions and the distances between these DNA regions.

More precisely, Figure 11 a illustrates an example of two distance thresholds $R_0$ and $R_1$ characterizing probabilities of contacts between DNA regions belonging to two different chromosomes of the same of one of different organisms and between DNA regions belonging to two different chromosomes of two different organisms, respectively.

According to a particular embodiment, the distance between DNA regions is a specific function that typically depends on a distance along a pair of DNA strands (which is particularly relevant for DNA regions of the same chromosome) and on a spatial distance (which is more relevant for DNA regions of different chromosomes).

Figure 11b is a graph representing the relation between the distance between two DNA regions (represented as the abscissa) and the contact probability between these two regions (represented as the ordinate). For the sake of illustration, it is considered that chromosomes are grouped per each of the organisms and virtually linked to each other into a single chain along which a distance between the DNA regions can be established.

As illustrated, if two DNA regions belong to the same chromosome, the distance between these two DNA regions is generally less than $R_0$ and the probability of contact between these two DNA regions is high as illustrated with the portion of curve 1100.

Similarly, if two DNA regions belong to two different chromosomes of a single one of the organisms, the distance between these two DNA regions is typically comprised between $R_0$ and $R_1$ and the probability of contact between these two DNA regions is medium as illustrated with the portion of curve 1105.

Finally, if two DNA regions belong to two different chromosomes of two different organisms, the distance between these two DNA regions is generally greater than $R_1$ and the probability of contact between these two DNA regions is low as illustrated with the portion of curve 1110.

In other words, the theoretical model aims at predicting contact frequencies as a function of two genomic positions. The illustrated theoretical model comprises three levels. A first level is directed to the intra-chromosomic contacts while a second level is directed to the intra-chromosomic and inter-chromosomic contacts and a third level is directed to contacts between different organisms.

The first and second levels are separated by $R_0$ value while the second and third levels are separated by $R_1$ value. The first level is based on the physics of polymer.

The parameters $R_0$ and $R_1$ are adjusted during execution of the GRAAL algorithm while exploring the corresponding distribution.

Of course, more than two thresholds $R_0$ and $R_1$ can be used according to, for example, properties that are specific to particular organisms.

According to a particular embodiment, the relation between probabilities $f$ of contacts between DNA regions $x$ and $y$ and the distances $d$ between these DNA regions can be expressed as follows:

$$f(x,y) = \begin{cases} M(x,y) \; if \; organism(x) = organism(y) \; and \; d \leq R_0 \\ C_1 \; if \; organism(x) = organism(y) \; and \; d > R_0 \\ C_2 \; if \; organism(x) \neq organism(y) \end{cases}$$

where $C_1$ and $C_2$ are constant values.

It is to be noted that the relation $f$ can be modified as follows so as to take into account a variability of contacts between compartments:

$$f(x,y) = \begin{cases} M(x,y) \; if \; organism(x) = organism(y) \; and \; d \leq R_0 \\ C_1 \; if \; organism(x) = organism(y) \; and \; d > R_0 \\ G(x,y) \; if \; organism(x) \neq organism(y) \end{cases}$$

More generally, this relation can be modified to be adapted to particular needs. For the sake of illustration, constants $C_1$ and $C_2$ that are used to predict intra and inter organism contacts can be replaced by and/or completed with other constants to take into account sub-assemblies such as symbiosis and intracompartmental reorganization.

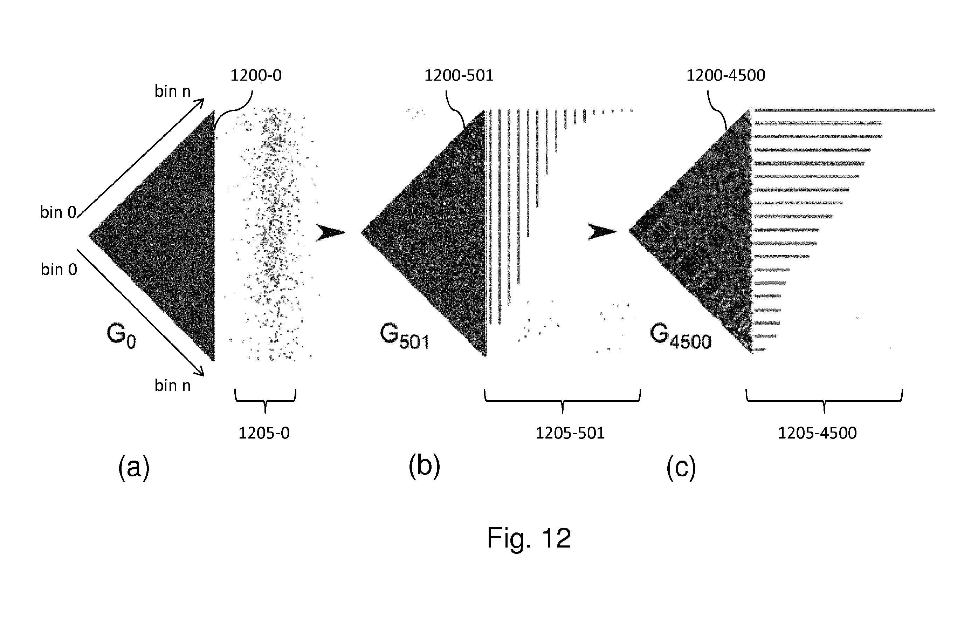

**Figure 12,** comprising Figure 12a, 12b, and 12c, illustrates an example of a half contact array between DNA fragments of a biological sample and of the corresponding genome structure, at three different iterations of the GRAAL algorithm ($t = 0$, $t = 501$, and $t = 4,500$).

The half contact array represents the number of contacts between two bins that are ordered as a function of their estimated position in the chromosome structure.

When the GRAAL algorithm is launched, the bins are pseudo-randomly ordered as illustrated in Figure 12a wherein the contact array 1200-0 and the chromosome structure 1205-0 do not represent any characteristic pattern.

After 501 iterations, some clusters begin to appear on the contact array 1200-501 and some patterns are identifiable on the chromosome structure 1205-501, as illustrated in Figure 12b.

Finally, after 4,500 iterations, clusters are clearly recognizable on the contact array 1200-4500 and the chromosome structure 1205-4500 shows structured items of information, as illustrated in Figure 12c.

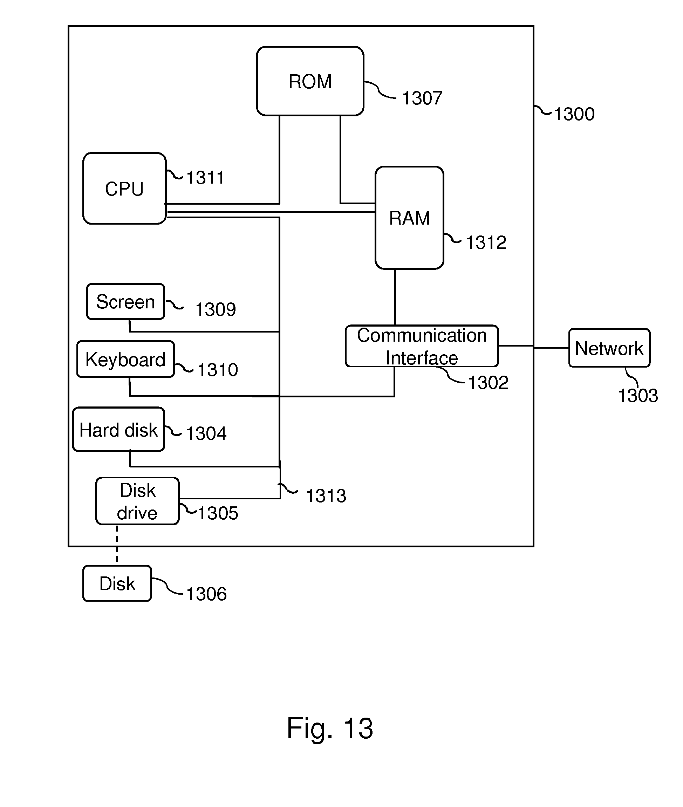

**Figure 13** schematically illustrates a processing device 1300 configured to implement at least one embodiment of at least part of the invention, for example one or several of the algorithms described by reference to Figures 6, 8, and 9. The processing device 1300 may be a device such as a micro-computer, a workstation, or a higly parallel computer. The device 1300 comprises a communication bus 1313 to which there are preferably connected:

- a central processing unit 1311, such as a microprocessor, denoted CPU;
- a read only memory 1307, denoted ROM, for storing computer programs for implementing the invention;

- a random access memory 1312, denoted RAM, for storing the executable code of the method of embodiments of the invention as well as the registers adapted to record variables and parameters necessary for implementing the method of determining a genome structure according to embodiments of the invention; and
- a communication interface 1302 connected to a communication network 1303 over which digital data to be processed can be transmitted.

Optionally, the apparatus 1300 may also include the following components:

- a data storage means 1304 such as a hard disk, for storing computer programs for implementing methods of one or more embodiments of the invention and data used or produced during the implementation of one or more embodiments of the invention;
- a disk drive 1305 for a disk 1306, the disk drive being adapted to read data from the disk 1306 or to write data onto said disk;
- a screen 1309 for displaying data and/or serving as a graphical interface with the user, by means of a keyboard 1310 or any other pointing means.

The communication bus provides communication and interoperability between the various elements included in the apparatus 1300 or connected to it. The representation of the bus is not limiting and in particular the central processing unit is operable to communicate instructions to any element of the apparatus 1300 directly or by means of another element of the apparatus 1300.

The disk 1306 can be replaced by any information medium such as for example a compact disk (CD-ROM), rewritable or not, a ZIP disk or a memory card and, in general terms, by an information storage means that can be read by a microcomputer or by a microprocessor, integrated or not into the apparatus, possibly removable and adapted to store one or more programs whose execution enables the method of encoding a sequence of digital images and/or the method of decoding a bitstream according to the invention to be implemented.

The executable code may be stored either in read only memory 1307, on the hard disk 1304 or on a removable digital medium such as for example a disk 1306 as described previously. According to a variant, the executable code of the programs can be received by means of the communication network 1303, via the interface 1302, in order to be stored in one of the storage means of the apparatus 1300 before being executed, such as the hard disk 1304.

The central processing unit 1311 is adapted to control and direct the execution of the instructions or portions of software code of the program or programs according to the invention, instructions that are stored in one of the aforementioned storage means. On powering up, the program or programs that are stored in a nonvolatile memory, for example on the hard disk 1304 or in the read only memory 1307, are transferred into the random access memory 1312, which then contains the executable code of the program or programs, as well as registers for storing the variables and parameters necessary for implementing the invention.

In this embodiment, the apparatus is a programmable apparatus which uses software to implement the invention. However, alternatively, the present invention may be implemented in hardware (for example, in the form of an Application Specific Integrated Circuit or ASIC).

It has been shown that chromosome 3D organization is correlated with metabolic state. Therefore, by characterizing the 3D organization of many species coexisting in a mix, it may unveil their metabolic state. This could be applied to deciphering the "growth" state of these species by comparison to each other, and to identify different stages during the evolution of the metapopulation (e.g. stress, rapid growth, quiescence, etc.).

In addition, this approach could be pushed towards the identification of symbiosis and parasitic events on the basis of the interactions between the chromosomes of the different species involved (potential interest in diagnostics experiments). Indeed, one can reasons that the more membrane surround the genetic material, the less likely materials present in different organelles are to interact during the 3C re-ligation step. In other words, the "noise" of the experiment would then be an indicator of the spatial proximity of the DNA fragments involved. The genome of parasites, for instance, could interact more with the host genome during an intracellular proliferation stage, such as in the vacuole of macrophages, than before the infection occurred, when the two genomes are more distant in space. Development of new cross-linking agents may be needed to increase the signal to noise ratio (towards noise). This application could be of importance for the development of diagnostic tools following invasive processes.

The disclosed method for assembling sequences representing pieces of chromosomes of one or more organisms can be used for identifying a genome of a eukaryotic cell, of a prokaryotic cell, or of a microorganism in a biological sample. In particular, the disclosed method can be used for identifying a genome of a microorganism in a biological sample, the microorganism being of one of the parasite, bacterium, archaea, fungi, yeast, and virus types. Those cells and microorganisms can be pathogenic, namely for plants or animals, or non-pathogenic. In a more particular embodiment, the biological sample contains or comprises more than one cell or microorganism species.

It is to be understood that if the GRAAL algorithm can be used with a Meta3C library, other types of library of DNA fragments can be used such as a Hi-C library or a combination of a shotgun library and a Hi-C library. More generally, the GRALL algorithm can be used with data representing DNA fragments and comprising items of information of contact or of proximity between some if these DNA fragments.

Naturally, in order to satisfy local and specific requirements, a person skilled in the art may apply to the solution described above many modifications and alterations all of which, however, are included within the scope of protection of the invention as defined by the following claims.

**Claims**

1. A method for computer for assembling at least one sequence representing at least one piece of at least one chromosome of at least one organism, based on a set of raw sub-sequences representing all DNA fragments of at least one library, the at least one library including DNA fragments comprising chains of contiguous nucleotides of the at least one chromosome and including DNA fragments comprising combinations of at least two chains of contiguous nucleotides of the at least one chromosome, the method comprising the steps of:

   - obtaining first values representing contact frequencies between DNA regions of the at least one chromosome, the first values being associated with second values representing distances between the corresponding DNA regions; and
   - iteratively carrying out the steps of:

      - updating a genome structure on the basis of the first and second values and on the basis of a theoretical model associating a contact probability between DNA regions with a distance between the corresponding DNA regions, the updated genome structure being representative of the real genome structure of the at least one piece of the at least one chromosome of the at least one organism; and
      - updating parameters of the theoretical model as a function of the updated genome structure.

2. The method according to claim 1 wherein a distance between two DNA regions is determined as a function of a distance between the two DNA regions along a predetermined path and/or a spatial distance between the two DNA regions.

3. The method according to claim 1 or claim 2 further comprising a step of splitting the raw sub-sequences representing all DNA fragments of at least one library into a plurality of bins.

4. The method according to any one of claims 1 to 3 further comprising a step of generating a plurality of genome candidate structures and of computing an explicit likelihood value for each of the generated candidate genome structures of being closer to the real genome structure.

5. The method according to claim 4 wherein the step of generating a plurality of genome candidate structures is based on predetermined structural variations comprising at least one variation among a translocation, a deletion, an inversion, and a duplication.

6. The method according to claim 4 or claim 5 wherein one of the generated genome candidate structures is selected as a function of the associated likelihood value according to a rule of the multiple try Metropolis type.

7. The method according to any one of claims 4 to 6, depending on claim 3, wherein genome candidate structures are determined by structural variations of bins.

8. The method according to any one of claims 1 to 7 wherein the step of updating the theoretical model parameters is based on an algorithm of the Gibbs sampler type.

9. The method according to any one of claims 1 to 8 wherein the theoretical model comprises at least one parameter representative of a threshold used to discriminate intra-chromosomic contacts between DNA regions from intra-chromosomic and inter-chromosomic contacts between DNA regions.

10. The method according to any one of claims 1 to 9 wherein the theoretical model comprises at least one parameter representative of a threshold used to discriminate intra-chromosomic contacts between DNA regions or intra-chromosomic and inter-chromosomic contacts between DNA regions from contacts between different organisms.

**11.** The method according to any one of claims 1 to 9 further comprising a step of clustering DNA fragments of the at least one library, each cluster being associated with a specific organism, the raw sub-sequences corresponding to the clustered DNA fragments being processed for sequencing on a cluster basis.

**12.** The method according to claim 11 wherein the step of clustering DNA fragments of the library is based on an algorithm of the Louvain type.

**13.** The method according to any one of claims 1 to 12 further comprising a step of identifying at least one DNA sequence in the at least one sequence representing the at least one piece of the at least one chromosome of the at least one organism.

**14.** The method according to any one of claims 1 to 12 for characterizing a global chromosome organization of at least one organism, further comprising a step of inferring a metabolic state of the at least one organism that global chromosome organization is characterized from the tridimensional organization of the corresponding genome.

**15.** A method for identifying a genome of an eukaryotic cell, of a prokaryotic cell, or of a microorganism in a biological sample, the method comprising each of the steps of the method for assembling at least one piece of at least one chromosome of at least one organism of any one of claims 1 to 14.

**16.** The method of claim 15 for identifying a genome of a microorganism in a biological sample, the microorganism being of one of the parasite, bacterium, archaea, fungi, yeast, and virus types.

**17.** The method according to any one of claims 1 to 16 further comprising the steps of:

- cross-linking pieces of chromosomes of a prepared biological sample comprising the at least one piece of the at least one chromosome;
- fragmenting the cross-linked chromosomes using restriction enzymes of at least two different types; and
- sequencing the fragments of chromosomes resulting from the fragmenting step.

**18.** A method for assembling at least one piece of at least one chromosome of at least one organism, the method comprising the steps of:

- preparing a biological sample comprising the at least one piece of the at least one chromosome;
- cross-linking pieces of chromosomes of the prepared biological sample;
- fragmenting the cross-linked chromosomes using restriction enzymes of at least two different types;
- sequencing the fragments of chromosomes resulting from the fragmenting step; and
- assembling the sequenced fragments of chromosomes.

**19.** An apparatus comprising means configured for carrying out each step of the method according to any one of claims 1 to 18.

**20.** A computer program product for a programmable apparatus, the computer program product comprising instructions for carrying out each step of the method according to any one of claims 1 to 16 when the program is loaded and executed by a programmable apparatus.

105-1

100

110-1
A C T C T A A T T    C A C A T A A C C

110-2
T G A G A T T A A    G T G T A T T G G
105-2

120-1    115    120-2

Fig. 1a
(prior art)

Fig. 1b
(prior art)

C T A A T T C A C A T T C A T A A

T C A G T A C T A A T T C A C A T

125

130

135

Fig. 1c
(prior art)

Fig. 2 (prior art)

Fig. 7

Fig. 3

Preparing a biological sample ⌐ 300

↓

Preparing Meta3C library ⌐ 305

↓

Determining genome structure and parameters ⌐ 310

↓

300 ⌐

Biological sample
(e.g. 30 mg of wet material)

↓

400 ⌐ Diluting (e.g. 150 mL of PBS)

↓

Cross-linking (e.g. 3% formaldehyde for
30 min at room temperature
and, next, 30 min at 4 °C)

405 ⌐

↓

Stopping reaction (e.g. adding Glycine 2,5 M,
i.e. 250 mM of the final concentration)

410 ⌐

↓ ⌐ 415

Collecting the fixed cells (e.g. centrifugation)
Cleaning (e.g. using 50 mL of PBS)
Collecting the fixed cells (e.g. centrifugation)
Freezing (e.g. -80 °C)

↓

Fig. 4

Defrosting pellets of cells on ice (e.g. 30 min) ⌐500

⌐505

Resuspending the defrosted cells (e.g. TE = Tris 10mM, EDTA 1mM)

Lysing the sample cells (e.g. Precellys, 3 cycles: 6700rpm, 3 x 20 sec on / 60 sec off) ⌐510

⌐515

Pooling lysed cells and treatening with SDS 10% (e.g. final concentration = 0,5%, incubating during 15 min at room temperature)

Dispatching the treated lysed cells in tubes ⌐520

⌐525-3

| Adding restriction buffer (e.g. 50µL), triton (e.g. 50µL triton X-100 10%), and restriction enzymes (e.g. 100 U MspI and water to obtain 500µL) | Adding restriction buffer (e.g. 50µL), triton (e.g. 50µL triton X-100 10%), and restriction enzymes (e.g. 100 U MluCI and water to obtain 500µL) | Adding restriction buffer (e.g. 50µL), triton (e.g. 50µL triton X-100 10%), and restriction enzymes (e.g. 100 U Sau3AI and water to obtain 500µL) |

525-1 ⌐          525-2 ⌐

Incubating the composition at enzyme activity temperature (e.g. during 3h) ⌐530

⌐535

Centrifuging the tubes (e.g. 16,000 RPM during 20 min)

Removing floating material and resuspending material of the bottom of the tube (e.g. in 500µL of water) ⌐540

⌐545

Pooling tubes with restriction enzymes (e.g. 3mL), adding ligation blending (e.g. 1,6mL of NEB ligation buffer, 160µL BSA 10mg/mL, 250 U of T4 DNA ligase, and water to obtain 16mL), and incubating (e.g. during 4h at 16°C)

Stopping reaction (e.g. adding EDTA (10mM final) and K proteinase (4mg) and incubating (e.g. during 12h at 65°C)) 550

Extracting DNA fragments (e.g. isopropanol and phenol chloroforme precipitating and next ethanol precipitating (resumption with Tris 10mM, e.g. 60µL) and RNAse treatment 560

305

Fig. 5

Fig. 6

Fig. 8

Fig. 9

1000

1005-1

1005-2

1005-3

(a)

1010-2

1010-4

1010-3

1010-1

(b)

1010'-rs

(d)

(c)

1010'-3

1010'-31

1010'-3n

Fig. 10

(e)

(a)

(b)

Fig. 11

Fig. 12

Fig. 13

Europäisches
Patentamt
European
Patent Office
Office européen
des brevets

# EUROPEAN SEARCH REPORT

Application Number

EP 14 30 5997

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NOAM KAPLAN ET AL: "High-throughput genome scaffolding from in vivo DNA interaction frequency", NATURE BIOTECHNOLOGY, vol. 31, no. 12, 24 November 2013 (2013-11-24), pages 1143-1147, XP055157787, ISSN: 1087-0156, DOI: 10.1038/nbt.2768 * abstract * * page 1143, left-hand column, paragraph 1 - page 1145, right-hand column, paragraph 1 * * page 1146, left-hand column, last paragraph - right-hand column, paragraph 1 * * figures 1-4 * | 1-20 | INV. G06F19/22 ADD. G06F19/24 |
| X | JOSHUA N BURTON ET AL: "Chromosome-scale scaffolding of de novo genome assemblies based on chromatin interactions", NATURE BIOTECHNOLOGY, vol. 31, no. 12, 3 November 2013 (2013-11-03), pages 1119-1125, XP055157783, ISSN: 1087-0156, DOI: 10.1038/nbt.2727 * abstract * * page 119, left-hand column, paragraph 2 - page 1121, left-hand column, paragraph 1 * * page 1123, left-hand column, paragraph 2 - page 1124, left-hand column, last paragraph * * figures 1-3 * | 1-20 | |
| | -/-- | | TECHNICAL FIELDS SEARCHED (IPC) G06F |

The present search report has been drawn up for all claims

| Place of search | Date of completion of the search | Examiner |
|---|---|---|
| Munich | 12 December 2014 | Hilbig, Matthias |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

........................................................................

& : member of the same patent family, corresponding
    document

EPO FORM 1503 03.82 (P04C01)

Europäisches
Patentamt

European
Patent Office

Office européen
des brevets

**EUROPEAN SEARCH REPORT**

Application Number

EP 14 30 5997

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIEBERMAN-AIDEN EREZ ET AL: "Comprehensive Mapping of Long-Range Interactions Reveals Folding Principles of the Human Genome", SCIENCE, AMERICAN ASSOCIATION FOR THE ADVANCEMENT OF SCIENCE, US, vol. 326, no. 5950, 1 October 2009 (2009-10-01), pages 289-293, XP002591649, ISSN: 0036-8075 * the whole document * | 1-20 | |
| A | AXEL COURNAC ET AL: "Normalization of a chromosomal contact map", BMC GENOMICS, vol. 13, no. 1, 1 January 2012 (2012-01-01), page 436, XP055157771, ISSN: 1471-2164, DOI: 10.1186/gb-2009-10-3-r25 * the whole document * | 1-20 | |
| A | DEKKER J ET AL: "Capturing Chromosome Conformation", SCIENCE, AMERICAN ASSOCIATION FOR THE ADVANCEMENT OF SCIENCE, US, vol. 295, 15 February 2002 (2002-02-15), pages 1306-1310, XP008104109, ISSN: 0036-8075, DOI: 10.1126/SCIENCE.1067799 * the whole document * | 1-20 | TECHNICAL FIELDS SEARCHED (IPC) |

The present search report has been drawn up for all claims

| Place of search | Date of completion of the search | Examiner |
|---|---|---|
| Munich | 12 December 2014 | Hilbig, Matthias |